# EUROPEAN PATENT SPECIFICATION

(11) **EP 3 469 349 B1**
(45) Date of publication and mention of the grant of the patent: **19.04.2023**
(21) Application number: 17731403.6
(22) Date of filing: 08.06.2017
(51) Int. Cl.: G01N 29/02, G01N 29/22, G01N 29/24, G01N 15/02

(54) **FLUID SENSOR ASSEMBLY**
FLUIDSENSORANORDNUNG
ENSEMBLE DE CAPTEUR DE FLUIDE

(30) Priority: 08.06.2016 US 201662347397 P
(43) Date of publication of application: 17.04.2019
(73) Proprietor: Eaton Intelligent Power Limited, Dublin 4 (IE)
(72) Inventor: GERARDI, Joseph, Dryden New York 13053 (US); INGRAM, Richard, Aurora New York 13026 (US); SLEDZIONA, Michael, Ithaca New York 14850 (US)
(74) Representative: Novagraaf Group
(86) International application number: PCT/US2017/036564
(87) International publication number: WO 2017/214403

(56) References cited:
- WO-A1-2012/031292
- WO-A1-2014/194379
- WO-A1-2016/083839
- US-A1- 2006 052 963
- US-A1- 2013 080 080
- US-A1- 2014 144 216

## Description

### TECHNICAL FIELD

The present disclosure generally relates to fluid systems, fluid sensors, and methods of monitoring fluid systems, such as lubrication systems associated with aircraft.

### BACKGROUND

This background description is set forth below for the purpose of providing context only. Therefore, any aspects of this background description, to the extent that it does not otherwise qualify as prior art, is neither expressly nor impliedly admitted as prior art against the instant disclosure.

Machinery may utilize lubrication systems that can be monitored in real-time for indication of mechanical and fluid degradation. As components of machinery wear, debris particles may break off and enter the lubrication system. Among other things, it may be desirable to determine the particle size distribution and material properties of any contamination in the fluid (e.g., the presence of debris particles). Some monitoring system designs may not be capable of detecting non-metallic wear debris particles. Larger debris particles may indicate more serious potential failure conditions, so a sampling strategy may not be appropriate for larger particles.

WO 2014/194379 A1 discloses a fluid sensor assembly as it is defined in the precharacterizing portion of claim 1. Further fluid sensor assemblies are shown in WO 2012/031292 A1, US 2013/080080 A1, US 2006/052963 A1, and US 2014/144216 A1.

An example of an acoustic monitoring system is disclosed in British Patent 1,012,010 (1963). U.S. Patent Nos. 4,381,674, 4,527,420, and 4,339,944 describe methods of ultrasonic particle sensing. Ultrasonic particle sensing is also described in "Evaluation of an On-Line Ultrasonic Particle Sensor Using Bearing Test Data", Nemarich, C.P., J.C. Tuner, and Whitesel, H.K., 41st Meeting of the Mechanical Failures Prevention Group, Patuxent River, MD (1986). U.S. Patent 6,205,848 describes a method and equipment for characterizing particles via acoustic signals. Utilizing ultrasonic transducers for wear debris measurements is descibed in (1) "Detection of precursor wear debris in lubrication systems", Edmonds, J., M. Resner, and K. Skharlet, IEEE, 2000; (2) "Helicopter/Tiltrotor Gearbox Debris Monitoring", Edmonds, J., J. Gerardi, G. Hickman, Navy SBIR Phase I IDI Final Report, 1995).

One or more of the above methods may be limited by the shape of the acoustic beam, which can lead to only a partial volume of the fluid that passes by the transducer being monitored. Particles outside the focus region, including larger size particles indicative of impending failure, might not be detected. Also, one or more of the above methods may not sample fast enough to detect all debris if the flow rate or debris concentration is relatively high. Moreover, some designs may be unable to reliably differentiate between metallic debris and non-metallic debris, air bubbles, or water.

In some designs, a transducer may send an interrogation pulse to a target zone and if a particle is encountered, it may deflect a signal back to the same transducer. Additionally, a transmit crystal ring-down may interfere with returns or echoes received back from targets that are very close to the transducer (e.g., an electrical signal from the transmit pulse may impinge on the time trace of the echo signal and this effect may increases with temperature). Further, with some designs, only small-angle signal deflection departures from the transducer axis may be received be the single transducer.

There is a desire for solutions/options that minimize or eliminate one or more shortcomings of fluid and monitoring systems. The foregoing discussion is intended only to illustrate examples of the present field and should not be taken as a disavowal of scope.

### SUMMARY

The present invention is a fluid sensor assembly as it is defined in claim 1 and a method of sensing particles in a fluid as it is defined in claim 10. The fluid sensor assembly includes a body including a fluid passage, a first sensor connected to the body and directed toward the fluid passage, and a second sensor connected to the body and directed toward the fluid passage. At least one of the first sensor and the second sensor is configured to transmit a signal into the fluid passage. At least one of the first sensor and the second sensor is configured to receive at least a deflected version of the signal. The signal may include an ultrasonic pulse. The first sensor may include a focused transmitting transducer and the second sensor may include a non-focused receiving transducer. The fluid passage includes a longitudinal axis.

A helical coil is disposed at least partially around the fluid passage and includes a longitudinal axis parallel to a longitudinal axis of the fluid passage. The first sensor is disposed at a first oblique angle relative to a longitudinal axis of the fluid passage and/or is disposed at a second oblique angle relative to a transverse axis of the body, and/or the first sensor and the second sensor are disposed in a V-shaped configuration. The first oblique angle may be between about 40 degrees and 50 degrees preferably between about 44 and about 46 degrees.The second sensor may be aligned with the transverse axis of the body. The first sensor may include a focused transmitting transducer. The first sensor may include a first crystal and the second sensor may include a second crystal.

In embodiments, the fluid sensor assembly may include a third sensor and a fourth sensor. The third sensor may be configured to transmit a second signal into the fluid passage and the fourth sensor may be configured to receive at least one of a deflected version of the second signal and the deflected version of the signal. The fluid sensor assembly may include a third sensor configured to receive the deflected version of the signal. The third sensor may be substantially coaxial with the second sensor. In embodiments, the fluid sensor assembly may include a fluid conduit connected to an inlet of the body. The fluid conduit may include a vertical portion, a bent portion, and/or an outlet. The outlet may be disposed at an oblique angle relative to a longitudinal axis of the fluid passage. The first sensor and the second sensor may be disposed in a V-shaped configuration.

The method of sensing particles in a fluid in accordance with the invention includes providing a fluid sensor assembly. The fluid sensor assembly includes a housing including a fluid passage, a first sensor connected to the housing and directed toward the fluid passage, a second sensor connected to the housing and directed toward the fluid passage, and a helical coil disposed at least partially around the fluid passage, the coil including a longitudinal axis parallel to a longitudinal axis of the fluid passage. The method further includes transmitting, via the first sensor, a signal into the fluid passage, receiving, via the second sensor, a deflected version of the signal that has deflected or scattered after encountering a debris particle, and detecting the debris particle according the deflected version of the signal. At least one of the first sensor and the second sensor is disposed at a first oblique angle relative to a longitudinal axis of the fluid passage and at a second oblique angle relative to a transverse axis of the housing. The method of sensing particles may include determining at least one of a size, a shape, and a material of the debris particle according to the deflected version of the signal. The method of sensing particles includes sensing the debris particle via an inductive sensor connected to the housing. The method of sensing particles may include transmitting, via the second sensor, a second signal into the fluid passage and/or receiving, via the first sensor, a deflected version of the second signal that has deflected or scatter after encountering a second debris particle. The method of sensing particles may include providing an electronic control unit (ECU), and/or applying, via the ECU, signal processing methods to information from the first sensor, information from the second sensor, and/or information from the inductive sensor to determine a size of the debris particle and/or whether the debris particle includes metal. The method of sensing particles may include transmitting, via the first sensor, a second signal into the fluid passage, and receiving, via the second sensor, a deflected version of the second signal that has deflected or scattered after encountering the debris particle.

Various aspects of the present disclosure will become apparent to those skilled in the art from the following detailed description of the various embodiments, when read in light of the accompanying drawings.

### BRIEF DESCRIPTION OF THE DRAWINGS

**FIG. 1** is a perspective view generally illustrating an embodiment of a fluid sensor assembly according to teachings of the present disclosure.
**FIGS. 2****,** **3 and 4** are cross-sectional views generally illustrating embodiments of fluid sensor assemblies according to teachings of the present disclosure.
**FIG. 5** is a perspective view generally illustrating an embodiment of a fluid sensor assembly according to teachings of the present disclosure.
**FIGS. 6 and 7** are cross-sectional views generally illustrating bodies of embodiments of fluid sensor assemblies according to teachings of the present disclosure.
**FIG. 8** is a side view generally illustrating an embodiment of a body of a fluid sensor assembly according to teachings of the present disclosure.
**FIG. 9** is a side view generally illustrating an embodiment of a sensor according to teachings of the present disclosure.
**FIG. 10** is a cross-section view generally illustrating an embodiment of a cartridge sensor according to teachings of the present disclosure.
**FIGS. 11** **and** **12** are cross-sectional views generally illustrating bodies of embodiments of fluid sensor assemblies according to teachings of the present disclosure.
**FIG. 13** is a side view generally illustrating embodiments of sensors according to teachings of the present disclosure.
**FIG. 14** is a side view generally illustrating an embodiment of a fluid conduit according to teachings of the present disclosure.
**FIG. 15** is a cross-sectional view generally illustrating an embodiment of a fluid conduit according to teachings of the present disclosure.

### DETAILED DESCRIPTION

Reference will now be made in detail to embodiments of the present disclosure, examples of which are described herein and illustrated in the accompanying drawings. While the present disclosure will be described in conjunction with embodiments and/or examples, it will be understood that they are not intended to limit the present disclosure to these embodiments and/or examples. On the contrary, the present disclosure is intended to cover alternatives, modifications and equivalents, which may be included within the scope of the present disclosure as defined by the appended claims.

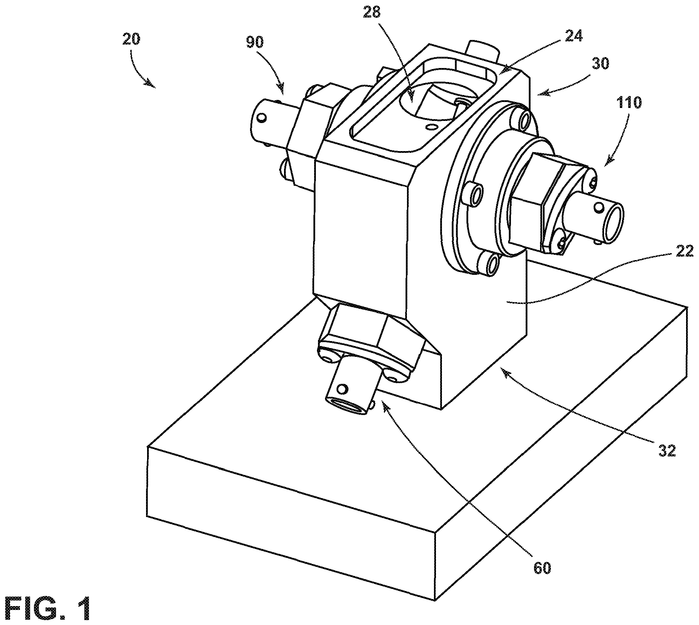

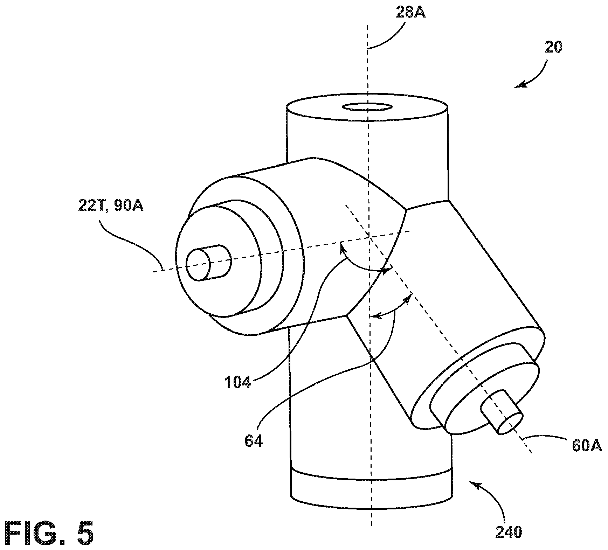

In embodiments, such as generally illustrated in **FIGS. 1****,** **2****,** **3, 4****, and** **5****,** a fluid sensor assembly 20 may include a body/housing 22 and a plurality of sensors, such as a first sensor 60, a second sensor 90, and/or a third sensor 110. Body 22 may include an inlet 24, an outlet 26, and/or a fluid passage 28 providing fluid communication between inlet 24 and outlet 26. Inlet 24 may be disposed at or about a first end 30 of body 22 (e.g., a top) and/or outlet 26 may be disposed at or about a second end 32 of body 22 (e.g., a bottom). Fluid passage 28 may include a longitudinal axis 28A that may, for example, be aligned and/or parallel with a vertical direction. Body 22 may, for example, be configured to hold or store, at least temporarily a volume of fluid 40. The fluid may contain particles 42. In embodiments, fluid sensor assembly 20 may be utilized in connection with a fluid separator, such as the fluid separator described in commonly owned U.S. Patent 7,288,139, which is hereby incorporated by reference herein in its entirety. For example and without limitation, debris particles 42 may be separated or at least directed into a particle receiving area and fluid sensor assembly 20 may be connected to the particle receiving area. Fluid 40 in body 22 may be relatively still (e.g. not flowing) during sensing, and particles 42 may fall within body 22 and bubbles may rise (e.g., due to gravity/density differences).

With embodiments, outlet 26 may be configured as a fluid exchange/renewal port that may allow for periodic exchange of fluid 40 within body 22, which may provide a more accurate and/or up-to-date sample of fluid 40 in a fluid system. Sensing may be suspended during fluid exchange. A fluid exchange may include a substantially complete purging of fluid 40 out of body 22 and new fluid 40 entering body (e.g., via inlet 24). A fluid exchange may involve some degree of backflow from outlet 26 to inlet 24 and may be gentle enough not to push larger particles 42 back through inlet and into the fluid system (e.g., back into a separator).

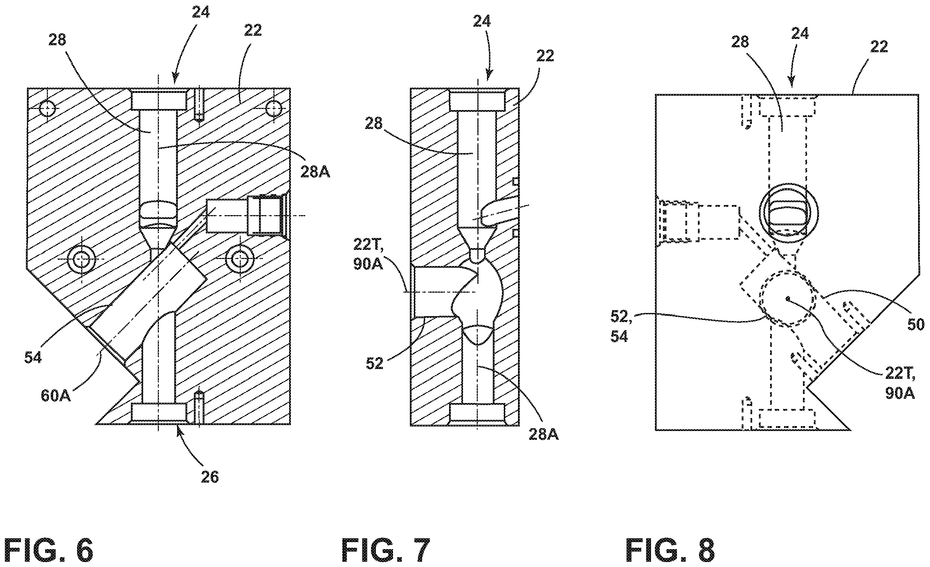

With embodiments, such as generally illustrated in **FIGS. 1-8****,** body 22 may include a first recess or aperture 50 configured to at least partially receive first sensor 60 and/or may include a second recess or aperture 52 configured to at least partially receive second sensor 90.

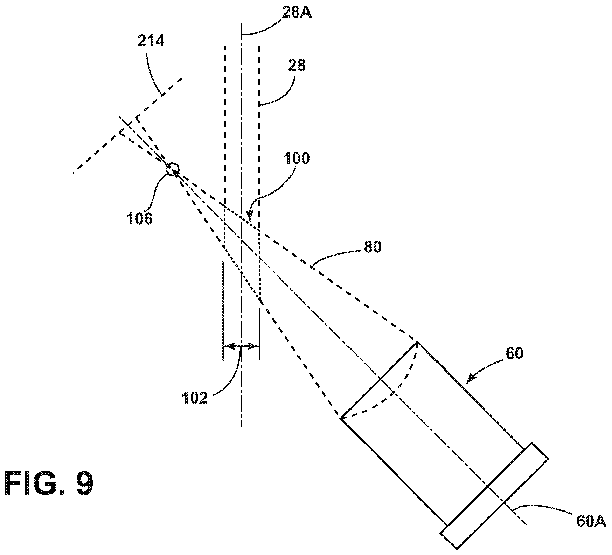

With embodiments, such as generally illustrated in **FIGS. 1-5****,** a sensor may include one or more of a variety of shapes, sizes, configurations, and/or materials. For example and without limitation, first sensor 60 may be configured to transmit a signal or signals 62 and/or second sensor 90 may be configured to receive a deflected or echoed version 92 of signal or signals 62. In embodiments, first sensor 60 may include a transmitting transducer configured to transmit (e.g., generate, propagate, emit, etc.) an acoustic signal/wave 62 toward and/or into fluid passage 28. For example and without limitation, first sensor 60 may include a piezoelectric crystal that may be pulsed at a frequency from about 2.5 MHz to about 5 MHz. The crystal may, for example, include a frequency of about 2.5 MHz or of about 5 MHz. First sensor 60 may be configured as a focused transducer, include a focused acoustic lens 72, and/or provide a focus zone 80 (see, e.g., **FIG. 9**). First sensor 60 and second sensor 90 may be disposed in a bistatic configuration. First sensor 60 may be connected with and/or at least partially disposed in first aperture 50 such that a first sensor axis 60A is disposed at angle 64 relative to longitudinal axis 28A. For example and without limitation, angle 64 may be an oblique angle relative to longitudinal axis 28A, such as an angle of about 40 degrees to 50 degrees, about 44 degrees to about 46 degrees, and/or about 45 degrees. A transverse axis 22T of body 22 may be perpendicular to longitudinal axis 28A. First sensor axis 60A may be aligned at an angle 66 (e.g., an oblique angle) relative to transverse axis 22T, for example and without limitation, an angle of about 40 degrees to 50 degrees, about 44 degrees to about 46 degrees, and/or about 45 degrees. First sensor axis 60A may intersect with longitudinal axis 28A at an intersection point 82 that may be disposed in fluid passage 28 (e.g., centered in passage 28). First sensor 60 may be directed generally toward first end 30 of body 22 (e.g., a first/transmit end 68 of first sensor 60 may be disposed closer to first end 30 of body 22 than an opposite end 70 of first sensor 60).

In embodiments, second sensor 90 may include a receiving transducer configured to receive acoustic signal/wave 62, and/or a version or portion thereof, transmitted by first sensor 60. For example and without limitation, first sensor 60 may transmit signal 62 in a first direction toward a target area or portion 100 of fluid passage 28. Target area 100 may be centered on longitudinal axis 28A and/or intersection point 82. If a particle 42 is present in the target portion 100, signal 62 may deflect off of the particle 42 and signal 62 may then be directed in one or more different directions, at least one direction of which may be toward second sensor 90. Second sensor 90 may be configured to receive a deflected version or portion 92 of signal 62 that has encountered a particle 42. Second sensor 90 may be configured to convert or translate a version or portion 92 of signal 62 received by second sensor 90 (e.g., an acoustic signal/wave) into an electrical signal that may correspond to one or more properties of the particle 42 (e.g., size, shape, etc.). With embodiments, second sensor 90 may be configured as a non-focused receiving transducer. In embodiments, second sensor 90 may be disposed as close to target area 100 as possible (e.g., at or just outside a drop diameter 102).

With embodiments, second sensor 90 may be connected to and/or at least partially disposed in second aperture 52 such that a second sensor axis 90A may be disposed in parallel with and/or may be coaxial with transverse axis 22T. Second sensor axis 90A may intersect with longitudinal axis 28A and/or first sensor axis 60A. For example and without limitation, longitudinal axis 28A, first sensor axis 60A, and second sensor axis 90A may pass through relatively small common area and/or may intersect at a common point (e.g., intersection point 82). Intersection point 82 may be disposed in target area or portion 100, such as at or about the center of target area or portion 100. First sensor 60 and second sensor 90 may be disposed at one or more angles relative to each other. For example and without limitation, first sensor 60 and second sensor 90 may be disposed at an angle 104 of about 90 degrees relative to each other (e.g., circumferentially/viewed along longitudinal axis 28A). In embodiments, a plurality of signals 62 may be transmitted and encounter (and deflect from) the same particle 42, which may provide additional information about the particle 42. First sensor and second sensor may be discrete, separate, and/or independent from each other.

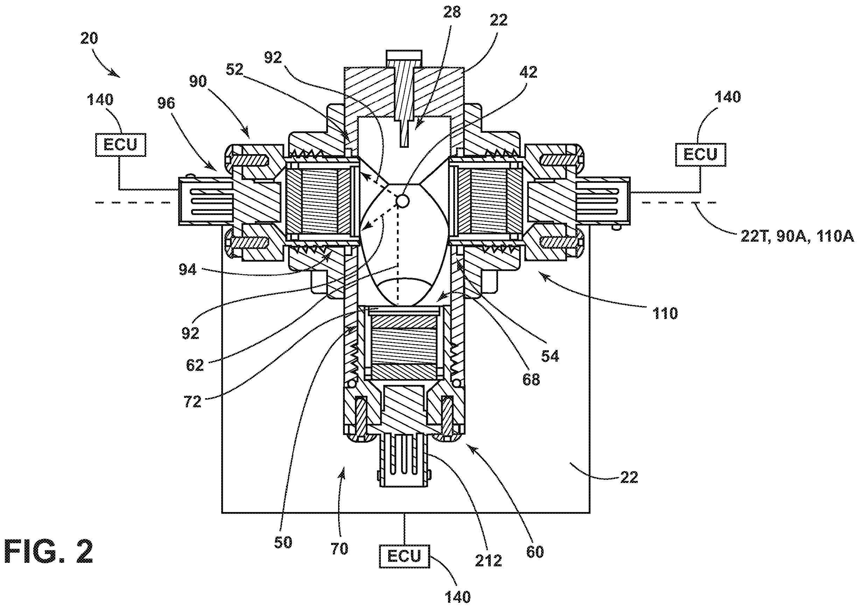

In embodiments, fluid sensor assembly 20 may include a third sensor 110 (see, e.g., **FIGS. 1** **and** **2**)**.** Body 22 may include a third recess 54 that may be configured for connection with and/or to at least partially receive third sensor 110. Third sensor 110 may be connected with and/or at least partially disposed in third recess 54 such that a third sensor axis 110A may be disposed substantially in parallel with transverse axis 22T, in parallel with second sensor axis 90A, and/or coaxial with second sensor axis 90A (e.g., second sensor 90 and third sensor 110 may be coaxial). Third sensor 110 may include a receiving transducer that may be configured to receive acoustic signals/waves 62. For example and without limitation, third sensor 110 may be configured to receive a deflected version or portion 92 of signal/wave 62 that has encounter/deflected off of a particle 42. A deflected version or portion 92 of signal/wave 62 received by third sensor 110 may have deflected in a different direction from particle 42 than a deflected version or portion 92 of signal/wave 62 received by second sensor 90. Third sensor 110 may be utilized in addition to and/or instead of second sensor 90. Utilizing third sensor 110 with second sensor 90 may allow for a greater portion of signal/wave 62 to be received after deflection, which may provide more or better information about a particle 42, especially for particles 42 of non-uniform shapes (e.g., non-spherical particles). For example and without limitation, if a particle 42 includes a facet and a deflected version 92 of signal 62 deflects to a relatively minimal extent or does not deflect toward second sensor 90, some of the deflected version 92 may be deflected to and/or received by third sensor 110 (or vice versa). With embodiments, third sensor 110 may be connected in parallel with second sensor 90.

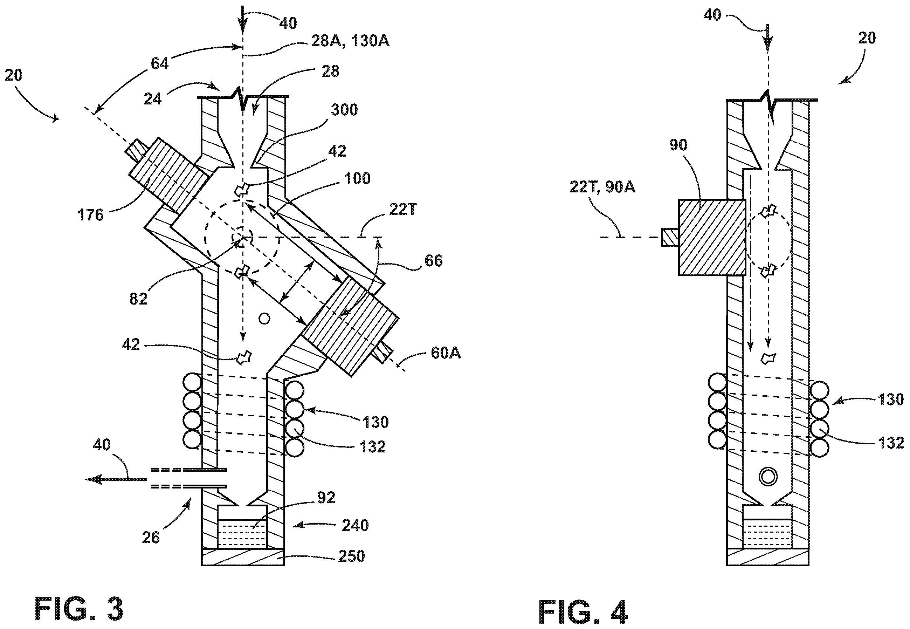

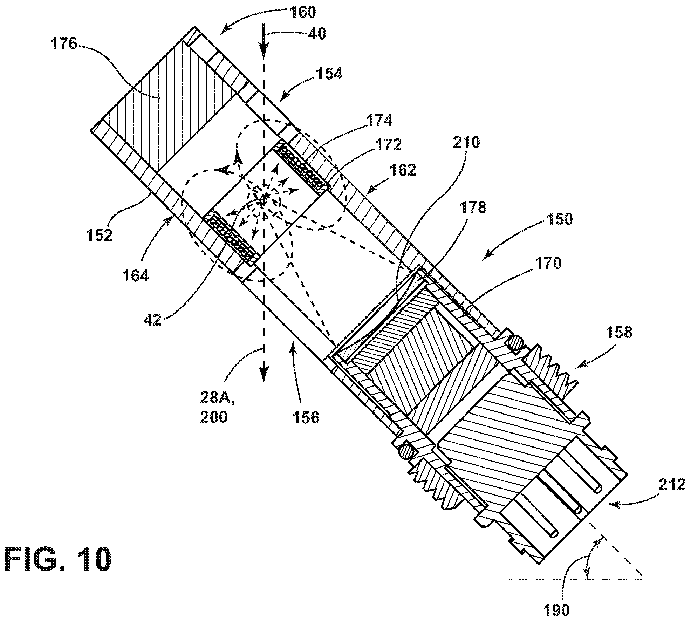

In embodiments, such as generally illustrated in **FIGS. 3, 4****,** **10****, and** **12****,** a fluid sensor assembly 20 may include a fourth sensor 130. Fourth sensor 130 may include an inductive sensor that may be configured to sense metallic particles 42 in body 22 and/or fluid passage 28. For example and without limitation, fourth sensor 130 may include a coil 132 that may be disposed around at least a portion of body 22 such that a fourth sensor axis 130A may be substantially aligned with (e.g., coaxial with) longitudinal axis 28A of fluid passage 28 (see, e.g., **FIGS. 3, 4****, and** **12**). Additionally or alternatively, a coil 132 may be disposed within body 22 and/or fluid passage 28 such that fourth sensor axis 130A is substantially aligned with longitudinal axis 28A of fluid passage 28 (see, e.g., **FIG. 10**). An electrical current may be provided to coil 132 and if a metal particle 42 moves through coil 132 (e.g., radially within coil 132), fourth sensor 130 may detect a change in inductance that may correspond to a size, shape, and/or material of the particle 42. Fourth sensor 130 may not be configured for detecting non-metal particles 42.

With embodiments, such as generally illustrated in **FIG. 2****,** an electronic control unit (ECU) 140 may be connected with one or more sensors (e.g., first sensor 60, second sensor 90, third sensor 110, fourth sensor 130, a cartridge sensor 150, a fifth sensor 230, a sixth sensor 270, and/or other sensors). ECU 140 may be configured to receive information (e.g., data, signals, etc.) from one or more sensors and ECU 140 may determine one or more of (i) whether particles 42 are present in fluid passage, (ii) a size associated with any such particle 42, (iii) a shape of any such particle 42, and/or (iv) a material of any such particle 42 (e.g., metal or non-metal). For example and without limitation, if a particle 42 is detected via first sensor 60 and second sensor 90 and is also detected by fourth sensor 130, ECU 140 may determine the particle 42 is metal. If a particle 42 is detected via first sensor 60 and second sensor 90, but is not detected by fourth sensor 130, ECU 140 may determine that the particle 42 is non-metal. ECU 140 may be configured to control operation of one or more sensors (e.g., first sensor 60, second sensor 90, third sensor 110, fourth sensor 130, cartridge sensor 150, fifth sensor 230, sixth sensor 270, and/or other sensors). ECU 140 may be configured to apply one or more signal processing methods to received information (e.g., information received from one or more of sensors 60, 90, 110, 130, 150, 230, 270). Signal processing methods may include various functions, for example and without limitation, averaging, fuzzy logic, and/or pattern recognition.

In embodiments, ECU 140 may include an electronic controller and/or include an electronic processor, such as a programmable microprocessor and/or microcontroller. In embodiments, ECU 140 may include, for example, an application specific integrated circuit (ASIC). ECU 140 may include a central processing unit (CPU), a memory, and/or an input/output (I/O) interface. ECU 140 may be configured to perform various functions, including those described in greater detail herein, with appropriate programming instructions and/or code embodied in software, hardware, and/or other medium. In embodiments, ECU 140 may include a plurality of controllers. In embodiments, ECU 140 may be connected to a display.

With embodiments, such as generally illustrated in **FIG. 10****,** a sensor 150 may include a cartridge configuration. A cartridge sensor 150 may include a body 152, a transmit transducer 170, a receiver 172, an inductive coil 174, a dirtiness sensor 176, and/or an acoustic lens 178. Body 152 may include a generally cylindrical or tubular shape that may be connected to sensor assembly body 22 at an angle, such as angle 190 (e.g., an oblique angle). Body 152 may include an inlet 154, an outlet 156, a first end 158, a second end 160, a first side 162 (e.g., a top), and/or a second side 164 (e.g., a bottom). Inlet 154 may include a slot or aperture in first side 162 and/or outlet 156 may include a slot or aperture in second side 164. Bubbles may purge via inlet 154. Inlet 154 and outlet 156 may be axially spaced from each other such that if body 152 is disposed at angle 190 (e.g., upon connection with body 22), a generally vertical fluid path 200 (e.g., a drop path) may be provided through body 152 from inlet 154 to outlet 156. Fluid path 28 of body 22 of fluid assembly 20 may at least partially align with and/or include fluid path 200 of body 152 of cartridge sensor 150.

In embodiments, transmitting transducer 170 may be disposed at or about first end 158 of body 152 and/or at least partially within body 152. Transmitting transducer 170 may be configured to transmit an acoustic signal/wave 62 into body toward second end 160 and/or may provide a focused acoustic field. Acoustic lens 178 may be disposed with body 152 at or about a transmitting end 158 of transmitting transducer 170 between transmitting transducer 170 and second end 160 of body 152. Particles 42 that move away from longitudinal axis 28A may fall onto transmitting transducer 170 and may slide down a tapered face 210 of transmitting transducer 170 and/or of acoustic lens 178 until the particles 42 reach outlet 156.

In embodiments, receiver 172 may include a generally ring-like or cylindrical configuration and may include, for example, a piezoelectric ring receiver (e.g., lead zirconate titanate or PZT) that may be configured to receive signals (e.g., deflected signals 92) from a plurality of directions. Receiver 172 may be disposed at least partially between inlet 154 and outlet 156 (e.g., axially) such that fluid path 200 may extend from inlet 154 through receiver 172 to outlet 156. For example and without limitation, fluid 40 and/or particles 42 may, at least initially, flow/fall into inlet 154, through receiver 172, and to outlet 156. Inductive coil 174 may be disposed at least partially between inlet 154 and outlet 156 (e.g., axially) such that fluid path 200 may extend from inlet 154 through inductive coil 174 to outlet 156. For example and without limitation, metal particles 42 that flow/fall into inlet 154 may fall through inductive coil 174, which may sense such metal particles 42, and metal particles 42 may continue to flow/fall to outlet 156. Inductive coil 174 may be coaxial with receiver 172 and/or inductive coil 174 and receiver 172 may be integrated and/or connected with each other. Dirtiness sensor 176 may be connected and/or disposed at or about second end 160 and may be directed toward first end 158, transmit transducer 170, and/or acoustic lens 178.

With embodiments, if fluid 40 is present in body 152 (e.g., if fluid 40 flows into body 152 through inlet 154), transmitting transducer 170 may transmit a signal 62 into body 152. If a particle 42 is present in body 152 and/or fluid 40, signal 62 may deflect from/off of the particle 42. Receiver 172 may receive at least a portion of the deflected signal 92 and may generate an electrical signal that may correspond to one or more characteristics of the particle 42 (e.g., size, shape, material, etc.). Receiver 172 may be connected to a connector 212 that may be connected at or about first end 158 of body 152 and/or may be connected to transmitting transducer 170. Connector 212 may, for example, be connected to ECU 140. If a metal particle 42 is present in or near inductive coil 174, an inductance of inductive coil 174 may change according to one or more characteristics of the particle 42 (e.g., size, shape). Additionally or alternatively, inductive coil 174 may be connected to connector 212 and/or ECU 140. With embodiments, cartridge sensor 150 may be connected with body 22 of fluid sensor assembly 20 and may be used in conjunction with and/or instead of first sensor 60, second sensor 90, and/or fourth sensor 130.

With embodiments, cartridge body 152 may include a generally square-shaped cross section. In such embodiments, receiver 172 and/or inductive coil 174 may include corresponding square-shaped cross sections.

In embodiments, a reverse configuration may include first sensor 60 including a receiving transducer and/or second sensor 90 including a transmitting transducer. For example and without limitation, second sensor 90 may transmit, without a focal zone, a signal 62 that may deflect from and/or off of a particle 42 and a deflected version or portion 92 of the signal 62 may be received by first sensor 60. In a reverse configuration, a transmitting transducer (e.g., second sensor 90) may be disposed closer to a target zone 100 (e.g., relative to first sensor 60 in a forward configuration), which may increase the energy of the transmit signal 62 that reaches a particle and/or the signal(s) 92 deflected/scattered from a particle 42. Additionally or alternatively, some of the acoustic energy that may have missed the receiving transducer (e.g., second sensor 90 in the forward configuration) may be at least indirectly received by first sensor 60 since signals 92 may deflect or scatter from a mounting pocket wall 214 opposite first sensor 60. A reverse configuration may be utilized, for example, to sense relatively small particles 42 (e.g., about 1,27µm - 2,54µm (50 µ to 100 µ) compared to 2,54µm - 25,4µm (100 µin to 1000 µin)). Sensing smaller particles 42 may be conducted via sampling as a full count of smaller particles may not be desired.

With embodiments, fluid sensor assembly 20 may be operated in both a bistatic sensor mode (e.g., for large particles) and a monostatic mode (e.g., for smaller particles, with first sensor 60 and/or second sensor 90 functioning as both a transmitting transducer and a receiving transducer). For example and without limitation, in one sensing cycle, a bistatic sensor mode may be used and then a monostatic mode may be used, and such a cycle may repeat. In a bistatic mode, ECU 140 may be configured to register multiple particles/hits on the same particle 42 such movement of a particle 42 can be tracked to distinguish particles from bubbles. If a large particle 42 is present during the monostatic mode, ECU 140 may identify the large particle based on its rate of descent and may ignore it (until the bistatic mode resumes).

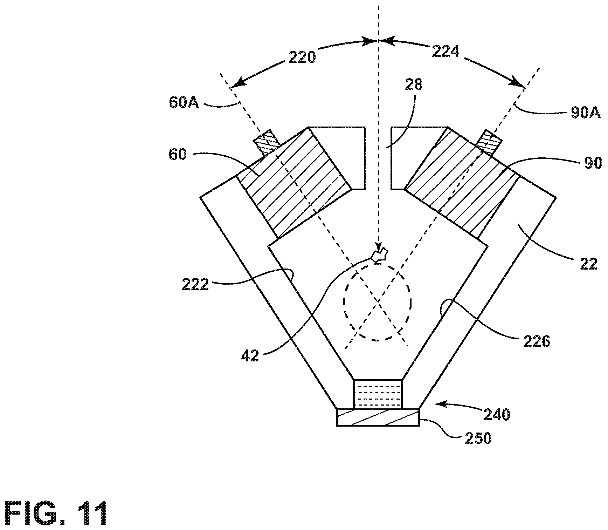

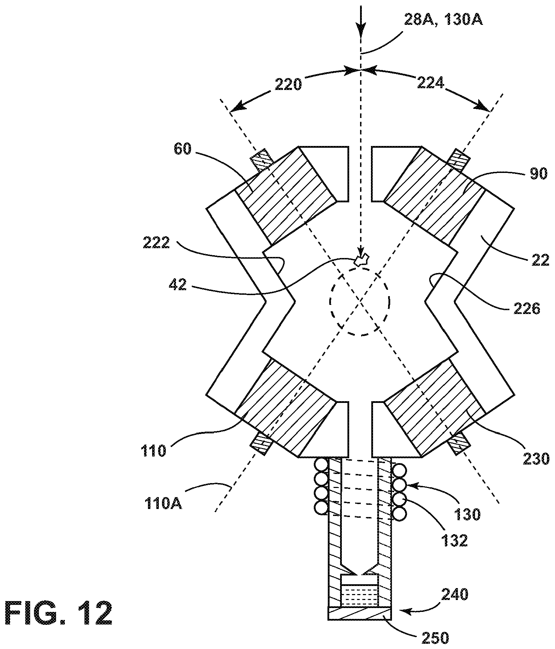

In embodiments, such as generally illustrated in **FIGS. 11** **and** **12****,** first sensor 60 and second sensor 90 may be disposed in a V-shaped configuration and/or body 22 may include a V-shaped configuration. For example and without limitation, first sensor 60 may be connected to body 22 such that first sensor axis 60A is disposed at a first acute angle 220 relative to longitudinal axis 28A, which may include first sensor 60 pointing generally downward with a transmitting end 68 of first sensor 60 closer to longitudinal axis 28A than an opposite end 70 of first sensor 60. A first inner wall 222 of body 22 may extend at first acute angle 220 and/or may be disposed opposite second sensor 90. Second sensor 90 may be connected to body 22 such that second sensor axis 90A may disposed at a second acute angle 224 relative to longitudinal axis 28A, which may include second sensor 90 pointing generally downward with a receiving end 94 of second sensor 90 closer to longitudinal axis 28A than an opposite end 96 of second sensor 90. A second inner wall 226 of body 22 may extend at second acute angle 224 and/or may be disposed opposite first sensor 60. First sensor 60 and second sensor 90 may be disposed at opposite sides of longitudinal axis 28A and may be substantially aligned in a common plane (e.g., a vertical plane). First acute angle 220 and second acute angle 224 may be substantially the same. For example and without limitation, first acute angle 220 and second acute angle 224 may be about 30 degrees to 40 degrees, about 33 degree to about 37 degrees, and/or about 35 degrees. An angle between first sensor axis 60A and second sensor axis 90A (e.g., a sum of angle and angle) may, for example, be about 70 degrees. In a V-shaped configuration, particles 42 may not accumulate at or near first sensor 60 and/or second sensor 90 (e.g., gravity may pull particles 42 generally downward/away from first sensor 60 and/or second sensor 90). A V-shaped configuration may provide a longer fluid path 28 (e.g., a drop path) within body 22 relative to other configurations, such as the configurations generally illustrated in **FIGS. 1-10****.** With embodiments, first sensor 60 and second sensor 90 may be disposed perpendicularly to fluid path axis 28A. For example and without limitation, first sensor axis 60A and second sensor axis 90A may be disposed at an angle relative to each other and/or be substantially aligned in the common plane (e.g., a horizontal plane).

With embodiments, fluid sensor assembly 20 may include an array of sensors. For example and without limitation, as generally illustrated in **FIG. 12****,** fluid sensor assembly 20 may include first sensor 60, second sensor 90, third sensor 110, fourth sensor 130, and/or a fifth sensor 230. Fifth sensor 230 may be configured in the same or a similar manner as first sensor 60. For example and without limitation, fifth sensor 230 may include a focused transmitting transducer configured to transmit an acoustic signal/wave 62 toward and/or into fluid passage 28. First sensor 60 and second sensor 90 may be disposed in a first V-configuration and third sensor 110 and fifth sensor 230 may be disposed in a second V-shaped configuration that may mirror the first V-configuration. For example and without limitation, first sensor 60, second sensor 90, third sensor 110, and fifth sensor 230 may be disposed in a X-shaped configuration. First sensor 60 and fifth sensor 230 may be substantially aligned with each other (e.g., coaxial) and/or second sensor 90 and third sensor 110 may be substantially aligned with each other. First sensor 60 and third sensor 110 may be disposed at a first side of longitudinal axis 28A and/or second sensor 90 and fifth sensor 230 may be disposed at a second side of longitudinal axis 28A. With embodiments, fifth sensor 230 may be used in other configurations, such as those generally illustrated in **FIGS. 1-5****.** For example and without limitation, fluid sensor assembly 20 may include one or more of first sensor 60, second sensor 90, third sensor 110, fourth sensor 130, and/or a fifth sensor 230.

In embodiments, such as generally illustrated in **FIGS. 3, 4****,** **5****,** **12****, and** **15****,** fluid sensor assembly 20 may include a debris port/pocket 240. Debris port 240 may be configured to receive and/or at least temporarily store particles 42 that flow/fall through fluid path 28. A cover 250 may be connected to debris port 240 and may permit access to debris particles 42 in debris port 240 (e.g., debris particles 42 may be removed for study). Debris port 240 and/or cover 250 may be magnetic.

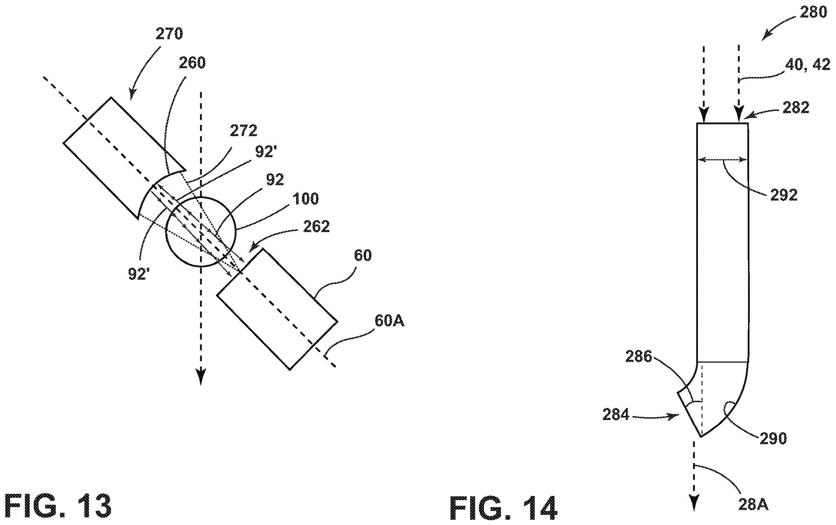

With embodiments, such as generally illustrated in **FIG. 13****,** fluid sensor assembly 20 may include a reflector 260 and a reverse configuration of first sensor and second sensor. Reflector 260 may be disposed opposite first sensor 60 (which may include/be configured as a receiving transducer). Reflector 260 may include a focus with a radius 262 that may extend to first sensor 60. Reflector 260 may be configured to reflect signals (e.g., signal/portion 92') toward first sensor 60. For example and without limitation, a signal 62 transmitted from second sensor 90 may result in two return signals 92, 92' reaching first sensor, such as an original, deflected signal 92 and a reflected signal 92'. The reflected signal 92' may be smaller than deflected signal 92 and/or may reach first sensor 60 after deflected signal 92. A time delay between deflected signal 92 and reflected signal 92' may be utilized to compensate for electrical noise. Additionally or alternatively, fluid sensor assembly 20 may include a sixth sensor 270 that may be disposed opposite first sensor 60. Sixth sensor 270 may include reflector 260 and/or may include a concave surface that may function as reflector 260. Sixth sensor may include a receiving transducer that may function as a trigger source to activate a listening mode of first sensor 60. The listening mode may correspond to a narrowly defined time window just before the arrival of a retro-reflection event from reflector 260 of sixth sensor 270. ECU 140 may sample a signal received by first sensor 60 to determine a peak in the signal. A field 272 of sixth sensor 270 may be low-noise or noise-free, such that deflected signal 92 and reflected signal 92' can be utilized and/or averaged, and ECU 140 may determine whether signals 92, 92' are a result of a bubble (if moving up) or a particle 42 (if falling/moving down).

With embodiments, such as generally illustrated in **FIG. 14****,** fluid sensor assembly 20 may include a fluid conduit 280 that may be connected to inlet 24 of body 22 and/or at least partially disposed in body 22. Fluid conduit 280 may include an inlet 282 may open substantially vertically upward and/or and outlet 284 that may open generally downward at an angle 286 relative to a vertical direction. For example and without limitation, fluid conduit 280 may include a bent section 288 that may bend at about 45 degrees and outlet 284 may open at an angle of about 45 degrees. If a particle 42 falls/flows from inlet 282 toward outlet 284, the particle 42 may contact an upward facing inner surface 290 of bent section 288 and may slide down surface 290 until the particle 42 falls out of outlet 284. Outlet 284 may be substantially aligned with longitudinal axis 28A of fluid passage 28 such that particles 42 exiting outlet 284 may fall substantially along longitudinal axis 28A, which may make sensing of particles 42 more consistent. Facilitating particles 42 falling along longitudinal axis 28A may reduce an expected drop zone 102 (e.g., a diameter around longitudinal axis 28A in which particles 42 are expected to fall, such as generally illustrated in **FIG. 9**), which may permit disposing sensors closer to longitudinal axis 28A (e.g., provide more energy to and/or receive more energy from particles 42) and/or allow for one or more sensors to include a semi-focus instead of a cylindrical field (or other shape). First sensor 60 may include a focal point or full focus zone 106. A fluid conduit 280 may be utilized in addition to and/or as an alternative to a constriction 300 at or about inlet 24 (see, e.g., **FIGS. 3 and 4**). Fluid conduit 280 may be less prone to clogging (e.g., relative to designs including constrictions) as an inner diameter 292 of fluid conduit 280 may be substantially constant from inlet 282 to outlet 284 (e.g., about 5.08 mm (0.2 inches)).

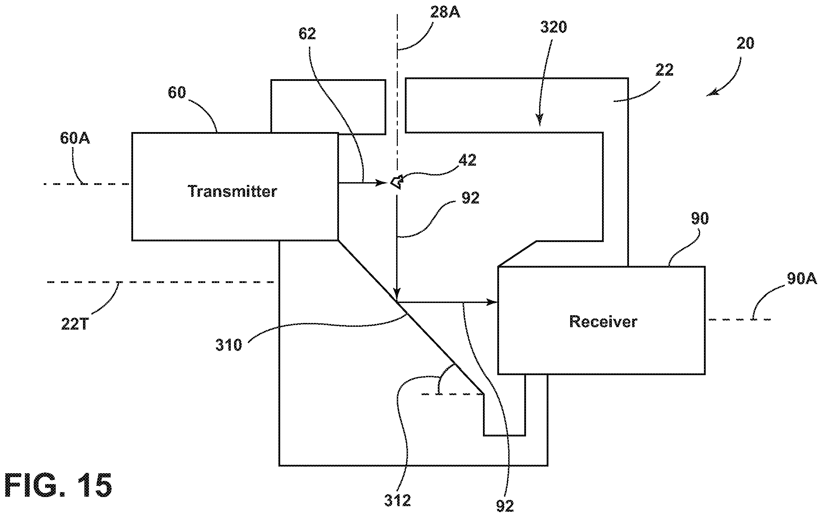

In embodiments, such as generally illustrated in **FIG. 15****,** body of fluid sensor assembly 20 may include a reflection wall 310 that may be aligned with longitudinal axis 28A (e.g., longitudinal axis 28A may intersect with tapered wall 310). First sensor 60 may include a transmitting transducer and may be disposed above reflection wall 310 such that first sensor axis 60A may be substantially parallel with a transverse axis 22T. Second sensor 90 may include a receiving transducer and/or be disposed such that second sensor axis 90A may be substantially parallel with transverse axis 22T and/or may intersect with reflection wall 310 at or about the same point as longitudinal axis 28A. Reflection wall 310 may include, for example, a taper angle 312 of about 45 degrees. In embodiments, reflection wall 310 may include a curvature and/or other shapes. First sensor 60 may transmit a signal 62 that may deflect off of or from a particle 42 and a deflected signal or portion 92 may reflect off of reflection wall 310 toward second sensor 90. Such a configuration may provide similar functionality as if second sensor 90 was mounted vertically upward and aligned with longitudinal axis 28A, but particles 42 may not accumulate on and/or obstruct second sensor 90.

In embodiments, a debris port 240 may be disposed below reflection wall 310. Particles 42 may fall until they reach reflection wall 310 and may slide down reflection wall 310 until the particles 42 fall into debris port 240.

With embodiments, body 22 may include a delay cavity 320 that may be disposed opposite first sensor 60. Signals 62 from first sensor 60 that pass beyond target zone 100 (e.g., significantly beyond longitudinal axis 28A) may enter delay cavity 320 and may become delayed enough and/or may attenuate enough not to contribute to signal noise.

With embodiments, a bistatic configuration of first sensor 60 and second sensor 90 may provide one or more advantages relative to other designs (e.g., monostatic designs). For example and without limitation, a ring-down of the transmit transducer may not be material as the transmit transducer (e.g., first sensor 60) may be acoustically isolated from the receiver (e.g., second sensor 90), which may allow for a larger gain to be used. The receiver (e.g., second sensor 90) may be disposed closer to the target zone 100 as it might be desirable to dispose a transmit transducer at a minimum distance from the target zone 100.

Various embodiments are described herein for various apparatuses, systems, and/or methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments.

Reference throughout the specification to "various embodiments," "with embodiments," "in embodiments," or "an embodiment," or the like, means that a particular feature, structure, or characteristic described in connection with the embodiment is included in at least one embodiment. Thus, appearances of the phrases "in various embodiments," "with embodiments," "in embodiments," or "an embodiment," or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features, structures, or characteristics of one or more other embodiments without limitation given that such combination is not illogical or non-functional.

It should be understood that references to a single element are not necessarily so limited and may include one or more of such element. Any directional references (e.g., plus, minus, upper, lower, upward, downward, left, right, leftward, rightward, top, bottom, above, below, vertical, horizontal, clockwise, and counterclockwise) are only used for identification purposes to aid the reader's understanding of the present disclosure, and do not create limitations, particularly as to the position, orientation, or use of embodiments.

Joinder references (e.g., attached, coupled, connected, and the like) are to be construed broadly and may include intermediate members between a connection of elements and relative movement between elements. As such, joinder references do not necessarily imply that two elements are directly connected/coupled and in fixed relation to each other. The use of "e.g." throughout the specification is to be construed broadly and is used to provide non-limiting examples of embodiments of the disclosure, and the disclosure is not limited to such examples. Uses of "and" and "or" are to be construed broadly (e.g., to be treated as "and/or"). For example and without limitation, uses of "and" do not necessarily require all elements or features listed, and uses of "or" are intended to be inclusive unless such a construction would be illogical.

It is intended that all matter contained in the above description or shown in the accompanying drawings shall be interpreted as illustrative only and not limiting. Changes in detail or structure may be made without departing from the present disclosure.

Furthermore, the mixing and matching of features, elements and/or functions between various examples is expressly contemplated herein so that one of ordinary skill in the art would appreciate from this disclosure that features, elements, and/or functions of one example may be incorporated into another example as appropriate, unless described otherwise, above. Moreover, many modifications may be made to adapt a particular situation or material to the teachings of the present disclosure without departing from the scope thereof. Therefore, it is intended that the present teachings not be limited to the particular examples illustrated by the drawings and described in the specification, but that the scope of the present disclosure will include any embodiments falling within the foregoing description and the appended drawings within the scope of the appended claims.

## Claims

1. A fluid sensor assembly (20), comprising:
a body (22) including a fluid passage (28);
a first sensor (60) connected to the body (22) and directed toward the fluid passage (28); and
a second sensor (90) connected to the body (22) and directed toward the fluid passage (28);
wherein at least one of the first sensor (60) and the second sensor (90) is configured to transmit a signal into the fluid passage (28), and at least one of the first sensor and the second sensor is configured to receive at least a deflected version of the signal;
the fluid sensor assembly further comprises an inductive sensor (130) comprising a helical coil (132) disposed at least partially around the fluid passage (28), the coil including a longitudinal axis (130A) parallel to a longitudinal axis (28A) of the fluid passage (28), the inductive sensor being configured for detecting metallic particles;
the fluid sensor assembly (20) further comprising an electronic control unit (ECU) (140) configured to receive and process data signals from the sensors (60, 90) and the inductive sensor (130) to determine particles in the fluid passage (28);
at least one of the following applies:
the first sensor (60) is disposed at a first oblique angle (64) relative to the longitudinal axis (28A) of the fluid passage (28);
the first sensor (60) is disposed at a second oblique angle (66) relative to a transverse axis (22T) of the body (22); and
the first sensor (60) and the second sensor (90) are disposed in a V-shaped configuration.

2. The fluid sensor assembly of claim 1, wherein the signal includes an ultrasonic signal, wherein the ultrasonic signal optionally includes a pulse.

3. The fluid sensor assembly of claim 1, wherein at least one of:
the first sensor (60) includes a focused transmitting transducer and the second sensor (90) includes a non-focused receiving transducer.; and
the first sensor (60) includes a first crystal and the second sensor (90) includes a second crystal.

4. The fluid sensor assembly of claim 3, wherein the first oblique angle (64) is between about 40 degrees and 50 degrees, preferably between about 44 and about 46 degrees.

5. The fluid sensor assembly of claim 3, wherein the second sensor (90) is aligned with the transverse axis (22T) of the body (22).

6. The fluid sensor assembly of claim 1, including a third sensor (110) and a fourth sensor (130), wherein the third sensor (110) is configured to transmit a second signal into the fluid passage (28) and the fourth sensor (130) is configured to receive at least one of a deflected version of the second signal and the deflected version of the signal.

7. The fluid sensor assembly of claim 1, comprising a third sensor (110) configured to receive the deflected version of the signal.

8. The fluid sensor assembly of claim 7, wherein the third sensor (110) is substantially coaxial with the second sensor (90) .

9. The fluid sensor assembly of claim 1, comprising a fluid conduit (280) connected to an inlet (24) of the body (22), the fluid conduit (280) including a vertical portion, a bent portion (288), and an outlet (284) disposed at an oblique angle (286) relative to a longitudinal axis (28A) of the fluid passage (28).

10. A method of sensing particles in a fluid, the method comprising:
providing a fluid sensor assembly (20), the fluid sensor assembly including:
a housing (22) including a fluid passage (28);
a first sensor (60) connected to the housing (22) and directed toward the fluid passage (28);
a second sensor (90) connected to the housing (22) and directed toward the fluid passage (28); and
an inductive sensor (130) comprising a helical coil (132) disposed at least partially around the fluid passage (28), the coil (132) including a longitudinal axis (130A) parallel to a longitudinal axis (28A) of the fluid passage (28) for detecting metallic particles;
transmitting, via the first sensor (60), a signal into the fluid passage (28);
receiving, via the second sensor (90), a deflected version of the signal that has deflected or scattered after encountering a debris particle and signals from the inductive sensor (130); and
detecting the debris particle according to the received signals;
wherein at least one of the first sensor (60) and the second sensor (90) is disposed at a first oblique angle (64) relative to the longitudinal axis (28A) of the fluid passage (28) and at a second oblique angle (66) relative to a transverse axis (22T) of the housing (22).

11. The method of claim 10, comprising determining, via an electronic control unit (140), at least one of a size, a shape, and a material of the debris particle according to the deflected version of the signal.

12. The method of claim 10, comprising sensing the debris particle via an inductive sensor connected to the housing (28).

13. The method of claim 10, comprising:
providing an electronic control unit (ECU) (140);
providing an inductive sensor; and
applying, via the ECU, signal processing methods to information from the first sensor (60), information from the second sensor (90), and information from the inductive sensor to determine a size of the debris particle and whether the debris particle includes metal.

14. The method of claim 10, comprising one of:
transmitting, via the second sensor (90), a second signal into the fluid passage (28); and receiving, via the first sensor (60), a deflected version of the second signal that has deflected or scattered after encountering a second debris particle; or
transmitting, via the first sensor (60), a second signal into the fluid passage (28); and receiving, via the second sensor (90), a deflected version of the second signal that has deflected or scattered after encountering the debris particle.

## Patentansprüche

1. Fluidsensoranordnung (20), umfassend:
einen Körper (22), der einen Fluiddurchlass (28) einschließt;
einen ersten Sensor (60), der mit dem Körper (22) verbunden und in Richtung des Fluiddurchlasses (28) gerichtet ist; und
einen zweiten Sensor (90), der mit dem Körper (22) verbunden und in Richtung des Fluiddurchlasses (28) gerichtet ist;
wobei mindestens einer des ersten Sensors (60) und des zweiten Sensors (90) konfiguriert ist, um ein Signal in den Fluiddurchgang (28) zu senden, und mindestens einer des ersten Sensors und des zweiten Sensors konfiguriert ist, um mindestens eine ausgelenkte Version des Signals zu empfangen;
wobei die Fluidsensoranordnung ferner einen induktiven Sensor (130) umfasst, umfassend eine spiralförmige Spule (132), die zumindest teilweise um den Fluiddurchlass (28) herum angeordnet ist, wobei die Spule eine Längsachse (130A) parallel zu einer Längsachse (28A) des Fluiddurchlasses (28) einschließt, wobei der induktive Sensor zum Erfassen von metallischen Partikeln konfiguriert ist;
die Fluidsensoranordnung (20) ferner umfassend eine elektronische Steuereinheit (ECU) (140), die konfiguriert ist, um Datensignale von den Sensoren (60, 90) und dem induktiven Sensor (130) zu empfangen und zu verarbeiten, um Partikel in dem Fluiddurchlass (28) zu bestimmen; und
mindestens eines aus Folgenden gilt:
der erste Sensor (60) ist in einem ersten schrägen Winkel (64) relativ zu der Längsachse (28A) des Fluiddurchlasses (28) angeordnet;
der erste Sensor (60) ist in einem zweiten schrägen Winkel (66) relativ zu einer Querachse (22T) des Körpers (22) angeordnet; und
der erste Sensor (60) und der zweite Sensor (90) sind in einer V-förmigen Konfiguration angeordnet.

2. Fluidsensoranordnung nach Anspruch 1, wobei das Signal ein Ultraschallsignal einschließt, wobei das Ultraschallsignal optional einen Impuls einschließt.

3. Fluidsensoranordnung nach Anspruch 1, wobei mindestens eines aus Folgenden gilt:
der erste Sensor (60) schließt einen fokussierten Sendewandler ein, und der zweite Sensor (90) schließt einen nicht fokussierten Empfangswandler ein; und
der erste Sensor (60) schließt einen ersten Kristall ein, und der zweite Sensor (90) schließt einen zweiten Kristall ein.

4. Fluidsensoranordnung nach Anspruch 3, wobei der erste schräge Winkel (64) zwischen etwa 40 Grad und 50 Grad, vorzugsweise zwischen etwa 44 und etwa 46 Grad liegt.

5. Fluidsensoranordnung nach Anspruch 3, wobei der zweite Sensor (90) mit der Querachse (22T) des Körpers (22) ausgerichtet ist.

6. Fluidsensoranordnung nach Anspruch 1, einschließlich eines dritten Sensors (110) und eines vierten Sensors (130), wobei der dritte Sensor (110) konfiguriert ist, um ein zweites Signal in den Fluiddurchlass (28) zu senden, und der vierte Sensor (130) konfiguriert ist, um mindestens eine von einer ausgelenkten Version des zweiten Signals und der ausgelenkten Version des Signals zu empfangen.

7. Fluidsensoranordnung nach Anspruch 1, umfassend einen dritten Sensor (110), der konfiguriert ist, um die ausgelenkte Version des Signals zu empfangen.

8. Fluidsensoranordnung nach Anspruch 7, wobei der dritte Sensor (110) im Wesentlichen koaxial mit dem zweiten Sensor (90) ist.

9. Fluidsensoranordnung nach Anspruch 1, umfassend eine Fluidleitung (280), die mit einem Einlass (24) des Körpers (22) verbunden ist, wobei die Fluidleitung (280) einen vertikalen Abschnitt, einen gebogenen Abschnitt (288) und einen Auslass (284) einschließt, der in einem schrägen Winkel (286) relativ zu einer Längsachse (28A) des Fluiddurchlasses (28) angeordnet ist.

10. Verfahren zum Erfassen von Partikeln in einem Fluid, das Verfahren umfassend:
Bereitstellen einer Fluidsensoranordnung (20), wobei die Fluidsensoranordnung Folgendes einschließt:
ein Gehäuse (22), das einen Fluiddurchlass (28) definiert;
einen ersten Sensor (60), der mit dem Gehäuse (22) verbunden und in Richtung des Fluiddurchlasses (28) gerichtet ist;
einen zweiten Sensor (90), der mit dem Gehäuse (22) verbunden und in Richtung des Fluiddurchlasses (28) gerichtet ist; und
einen induktiven Sensor (130) umfassend eine spiralförmige Spule (132), die zumindest teilweise um den Fluiddurchlass (28) herum angeordnet ist, wobei die Spule (132) eine Längsachse (130A) parallel zu einer Längsachse (28A) des Fluiddurchlasses (28) zum Erfassen von metallischen Partikeln einschließt;
Senden, über den ersten Sensor (60), eines Signals in den Fluiddurchlass (28);
Empfangen, über den zweiten Sensor (90), einer ausgelenkten Version des Signals, das nach dem Treffen auf ein Fremdkörperpartikel und Signale von dem induktiven Sensor (130) ausgelenkt oder gestreut wurde; und
Erfassen des Fremdkörperpartikels gemäß den empfangenen Signalen;
wobei mindestens einer des ersten Sensors (60) und des zweiten Sensors (90) in einem ersten schrägen Winkel (64) relativ zu der Längsachse (28A) des Fluiddurchlasses (28) und in einem zweiten schrägen Winkel (66) relativ zu einer Querachse (22T) des Gehäuses (22) angeordnet ist.

11. Verfahren nach Anspruch 10, umfassend das Bestimmen, über eine elektronische Steuereinheit (140), mindestens eines von einer Größe, einer Form und einem Material des Fremdkörperpartikels gemäß der ausgelenkten Version des Signals.

12. Verfahren nach Anspruch 10, umfassend das Erfassen des Fremdkörpertpartikels über einen induktiven Sensor, der mit dem Gehäuse (28) verbunden ist.

13. Verfahren nach Anspruch 10, umfassend:
Bereitstellen einer elektronischen Steuereinheit (ECU) (140);
Bereitstellen eines induktiven Sensors; und
Anwenden, über die ECU, von Signalverarbeitungsverfahren auf Informationen von dem ersten Sensor (60), Informationen von dem zweiten Sensor (90) und Informationen von dem induktiven Sensor, um eine Größe des Fremdkörperpartikels zu bestimmen und ob das Fremdkörperpartikel Metall enthält.

14. Verfahren nach Anspruch 10, ferner umfassend eines aus Folgenden:
Senden, über den zweiten Sensor (90), eines zweiten Signals in den Fluiddurchlass (28); und Empfangen, über den ersten Sensor (60), einer ausgelenkten Version des zweiten Signals, das nach dem Treffen auf ein zweites Fremdkörperpartikel ausgelenkt oder gestreut wurde; oder
Senden, über den ersten Sensor (60), eines zweiten Signals in den Fluiddurchlass (28); und Empfangen, über den zweiten Sensor (90), einer ausgelenkten Version des zweiten Signals, das nach dem Treffen auf das Fremdkörperpartikel ausgelenkt oder gestreut wurde.

## Revendications

1. Ensemble capteur de fluide (20), comprenant :
un corps (22) comportant un passage de fluide (28) ;
un premier capteur (60) relié au corps (22) et dirigé vers le passage de fluide (28) ; et
un deuxième capteur (90) relié au corps (22) et dirigé vers le passage de fluide (28) ;
au moins un parmi le premier capteur (60) et le deuxième capteur (90) étant conçu pour transmettre un signal dans le passage de fluide (28), et au moins un parmi le premier capteur et le deuxième capteur étant conçu pour recevoir au moins une version déviée du signal ;
l'ensemble capteur de fluide comprend en outre un capteur inductif (130) comprenant une bobine hélicoïdale (132) disposée au moins partiellement autour du passage de fluide (28), la bobine comportant un axe longitudinal (130A) parallèle à un axe longitudinal (28A) du passage de fluide (28), le capteur inductif étant conçu pour détecter des particules métalliques ;
l'ensemble capteur de fluide (20) comprenant en outre une unité de commande électronique (ECU) (140) configurée pour recevoir et traiter des signaux de données provenant des capteurs (60, 90) et du capteur inductif (130) pour déterminer des particules dans le passage de fluide (28) ; et
au moins l'un des éléments suivants s'applique :
le premier capteur (60) est disposé selon un premier angle oblique (64) par rapport à l'axe longitudinal (28A) du passage de fluide (28) ;
le premier capteur (60) est disposé selon un second angle oblique (66) par rapport à un axe transversal (22T) du corps (22) ; et
le premier capteur (60) et le deuxième capteur (90) sont disposés dans une configuration en forme de V.

2. Ensemble capteur de fluide selon la revendication 1, dans lequel le signal comporte un signal ultrasonore, dans lequel le signal ultrasonore comporte éventuellement une impulsion.

3. Ensemble capteur de fluide selon la revendication 1, dans lequel au moins un parmi :
le premier capteur (60) comporte un transducteur de transmission focalisé et le deuxième capteur (90) comporte un transducteur de réception non focalisé ; et
le premier capteur (60) comporte un premier cristal et le deuxième capteur (90) comporte un second cristal.

4. Ensemble capteur de fluide selon la revendication 3, dans lequel le premier angle oblique (64) est compris entre environ 40 degrés et 50 degrés, de préférence entre environ 44 et environ 46 degrés.

5. Ensemble capteur de fluide selon la revendication 3, dans lequel le deuxième capteur (90) est aligné avec l'axe transversal (22T) du corps (22).

6. Ensemble capteur de fluide selon la revendication 1, comportant un troisième capteur (110) et un quatrième capteur (130), dans lequel le troisième capteur (110) est conçu pour transmettre un second signal dans le passage de fluide (28) et le quatrième capteur (130) est conçu pour recevoir au moins l'une parmi une version déviée du second signal et la version déviée du signal.

7. Ensemble capteur de fluide selon la revendication 1, comprenant un troisième capteur (110) conçu pour recevoir la version déviée du signal.

8. Ensemble capteur de fluide selon la revendication 7, dans lequel le troisième capteur (110) est sensiblement coaxial avec le deuxième capteur (90).

9. Ensemble capteur de fluide selon la revendication 1, comprenant un conduit de fluide (280) relié à une entrée (24) du corps (22), le conduit de fluide (280) comportant une partie verticale, une partie pliée (288) et une sortie (284) disposée selon un angle oblique (286) par rapport à un axe longitudinal (28A) du passage de fluide (28).

10. Procédé de détection de particules dans un fluide, le procédé comprenant :
la fourniture d'un ensemble capteur de fluide (20), l'ensemble capteur de fluide comportant :
un boîtier (22) comportant un passage de fluide (28) ;
un premier capteur (60) relié au boîtier (22) et dirigé vers le passage de fluide (28) ;
un deuxième capteur (90) relié au boîtier (22) et dirigé vers le passage de fluide (28) ; et
un capteur inductif (130) comprenant une bobine hélicoïdale (132) disposée au moins partiellement autour du passage de fluide (28), la bobine (132) comportant un axe longitudinal (130A) parallèle à un axe longitudinal (28A) du passage de fluide (28) pour détecter des particules métalliques ;
la transmission, par l'intermédiaire du premier capteur (60), d'un signal dans le passage de fluide (28) ;
la réception, par l'intermédiaire du deuxième capteur (90), d'une version déviée du signal qui a été dévié ou diffusé après avoir rencontré une particule de débris et des signaux provenant du capteur inductif (130) ; et
la détection de la particule de débris selon les signaux reçus ;
au moins l'un parmi le premier capteur (60) et le deuxième capteur (90) étant disposé selon un premier angle oblique (64) par rapport à l'axe longitudinal (28A) du passage de fluide (28) et selon un second angle oblique (66) par rapport à un axe transversal (22T) du boîtier (22).

11. Procédé selon la revendication 10, comprenant la détermination, par l'intermédiaire d'une unité de commande électronique (140), d'au moins un parmi une taille, une forme et un matériau de la particule de débris selon la version déviée du signal.

12. Procédé selon la revendication 10, comprenant la détection de la particule de débris par l'intermédiaire d'un capteur inductif connecté au boîtier (28).

13. Procédé selon la revendication 10, comprenant :
la fourniture d'une unité de commande électronique (ECU) (140) ;
la fourniture d'un capteur inductif ; et
l'application, par l'intermédiaire de l'ECU, de procédés de traitement de signal à des informations provenant du premier capteur (60), d'informations provenant du deuxième capteur (90) et d'informations provenant du capteur inductif pour déterminer une taille de la particule de débris et si la particule de débris comporte du métal.

14. Procédé selon la revendication 10, comprenant une parmi :
la transmission, par l'intermédiaire du deuxième capteur (90), d'un second signal dans le passage de fluide (28) ; et la réception, par l'intermédiaire du premier capteur (60), d'une version déviée du second signal qui a été dévié ou diffusé après avoir rencontré une seconde particule de débris ; ou
la transmission, par l'intermédiaire du premier capteur (60), d'un second signal dans le passage de fluide (28) ; et la réception, par l'intermédiaire du deuxième capteur (90), d'une version déviée du second signal qui a été dévié ou diffusé après avoir rencontré la particule de débris.
